# EUROPEAN PATENT SPECIFICATION

(11) **EP 1 727 516 B1**
(45) Date of publication and mention of the grant of the patent: **14.05.2014**
(21) Application number: 05732360.2
(22) Date of filing: 23.03.2005
(51) Int. Cl.: A61K 8/36, A61K 8/37, A61K 8/67, A61Q 7/00, A61Q 19/02, A61Q 19/08

(54) **COMPOSITION COMPRISING AN HDAC INHIBITOR IN COMBINATION WITH A RETINOID**
EINEN HDAC-INHIBITOR IN KOMBINATION MIT EINEM RETINOID ENTHALTENDE ZUSAMMENSETZUNG
COMPOSITION COMPRENANT UN INHIBITEUR D'HDAC COMBINE A UN RETINOIDE

(30) Priority: 26.03.2004 EP 04007281
(43) Date of publication of application: 06.12.2006
(73) Proprietor: DSM IP Assets B.V., 6411 TE Heerlen (NL)
(72) Inventor: SCHEHLMANN, Volker, 79650 Schopfheim (DE); KLOCK, Jochen, 79104 Freiburg (DE); MAILLAN, Philippe, Emmanuel, F-68440 Eschentzwiller (FR); VOLLHARDT, Juergen, H., CH-4433 Ramlinsburg (CH); RAWLINGS, Anthony, Northwich Cheshire CW9 8FH (GB); BEUMER, Raphael, 79541 Loerrach (DE)
(74) Representative: Best, Michael
(86) International application number: PCT/EP2005/003115
(87) International publication number: WO 2005/092283

(56) References cited:
- EP-A- 1 491 188
- EP-A- 1 508 328
- WO-A-97/27836
- WO-A-02/060430
- US-A1- 2002 119 114
- US-A1- 2004 018 958
- US-B1- 6 365 623

## Description

The present invention is directed to topical compositions, which contain a combination of at least one histone deacetylase inhibitor (HDAC inhibitor) and a retinoid. The topical composition is preferably a cosmetic preparation. It was found that the combination of a HDAC inhibitor and a retinoid is in particular useful for treating wrinkles but also for thickening the epidermis.

Together, histone acetyltransferases (HATs) and histone deacetylases (HDACs) determine the acetylation status of histones. The acetylation affects the regulation of gene expression. Inhibitors of HDACs have been found to alter the transcription of a small number of genes. Histone proteins organize DNA into nucleosomes, which are regular repeating structures of chromatin. The nucleosome contains 146 bp of DNA wrapped around the core histone octamer. The histone octamer is composed of a H3/H4 tetramer and two H2A/H2B dimers. These proteins are very basic and highly conserved throughout evolution. All 4 core histones have an amino-terminal tail. This is lysine rich and contains about half of the positively charged residues and most of the post-translational modification sites of these histones. The N-terminal tail of the histone passes through and around of the enveloping DNA double helix. It has been proposed that there is a 'histone code' or 'epigenetic code' defined by specific modifications of the N-terminal tails such as deacetylation / acetylation, that regulate the transcriptional activity of specific genes. This modification is needed for multiprotein complexes to form the transcription-activating and transcription-repressing molecular machinery.

The acetylation status of chromatin is dependent on both HAT and HDAC activity. HDACs are involved primarily in the repression of gene transcription by virtue of the compaction of chromatin structure that accompanies the removal of charge neutralizing acetyl groups from the histone N-terminal tails.

In humans 3 classes of HDAC have been identified that are mainly divided by their structural homology and might be involved in modeling the structure of chromatin. Class I HDACs includes HDAC1, HDAC2, HDAC3 and HDAC8 whereas class II includes HDAC4, HDAC5, HDAC6, HDAC7, HDAC9 and HDAC10. HDAC class I and II map each to different chromosomal sites and are known to be sensitive for inhibitors. The third class of HDACs is the conserved SIR2 family of proteins (SIRT 1 to 7) that are absolute dependent on NAD⁺ for activity and are insensitive to known inhibitors.

HDAC inhibitors are thought to prolong the hyperacetylated status or induce the hyperacetylation of specific genes via the class I and II enzymes and by the competition between HDAC and HAT to the nucleosomes. By that HDAC inhibitors are inducing or stimulating the transcription of specific genes. Inhibitors of HDACs are proving to be an exciting therapeutic approach to cancer as it was found that they cause growth arrest, differentiation and apoptosis of many tumor cells by altering the transcription of a small number of genes. One good overview of presently known HDAC inhibitors can be found in Expert Opin. Ther. Patents 2002, 12 (9), pages 1375-1384.

WO 98/00127 discloses certain retinoyloxy-substituted alkylene butyrates and pharmaceutically acceptable salts thereof which are said to be suitable for treating cancer and other proliferative diseases. Furthermore, these compounds are said to be useful in methods of inhibiting histone deacetylase, ameliorating wrinkles, treating or ameliorating dermatological disorders, inducing wound healing, treating cutaneous ulcers and treating gastrointestinal disorders. A combination of butyric acid and retinoic acid is said to be known as differentiating or anti-proliferating agent for the treatment of cancer. Combinations of a HDAC inhibitor with retinol are not disclosed.

Human skin undergoes certain normal cornification processes, which give the skin its characteristic appearance. Casual factors or external factors such as a raw climate, wind, photodamage and irritation triggered by the sun, rain and snow, however, disturb this normal condition of the skin, and there appears a roughness, a formation of scales (for example on the scalp), an excessive keratinization and similar phenomena. Furthermore, in the course of aging of the skin various signs appear that are especially reflected by a change in the structure and function of the skin. One of these signs is the appearance of fine lines and deep wrinkles, the size and number of which increase with age. The microrelief of the skin becomes less uniform and is of unisotropic nature. In parallel with age the skin becomes more sensitive towards disturbing influences, either intrinsic or extrinsic, which may result in itch, redness or even darker spots, particular on hands and the facial area due to pigmentation disorders. These unwanted signs may lead to an undesired age judgment of a person.

Cosmetic preparations are essentially useful for skin care. One aim of skin care in the cosmetic sense is to strengthen or rebuild the skin's natural function as a barrier against environmental influences (e.g. UV-light, dirt, chemicals and microorganisms) and against the loss of endogenous substances (e.g. water, natural lipids, electrolytes). If this function becomes impaired, increased resorption of toxic or allergenic substances or attack by microorganisms may result, leading to toxic or allergic skin reactions.

Another aim of skin care is to compensate for the loss by the skin of lipids and water caused by daily washing. This is particularly important, if the natural regeneration ability is inadequate. Furthermore, skin care products should protect against environmental influences, in particular against sun and wind, and delay skin aging.

Strengthening or thickening of the epidermis together with an optimized skin barrier lipid synthesis can rebuild the skin's barrier ability and is therefore of significant cosmetic value. Reduced transepidermal water loss (TEWL) is a sign for an intact lipid barrier. Such an intact barrier acts also as first defense line to protect against the appearance of skin wrinkles.

Of particular importance for anti-aging cosmetics is to inhibit the senescence of skin cells in order to keep their regular metabolic level on a constant and beneficial level.

Another strategy to fight wrinkles is to stimulate the collagen synthesis in the dermis. A number of degradative processes act on the collagen matrix and is triggered by extrinsic factors like UV radiation, pollution in general and particular cigarette smoke or intrinsic factors like chronic and subchronic inflammation. Destruction and/or impaired repair efficacy leads to a denser and less elastic macro structure of the dermis, which in turn leads to the formation of deep wrinkles. Enhancing the *de novo* synthesis of collagen or other structural proteins of the dermis is considered a valuable therapy to reduce the existing wrinkles and to protect against the appearance of new wrinkles.

Retinoids are known to stimulate collagen synthesis and also to modulate degradative processes for example by reducing the expression of matrix metallo proteases (MMPs) at a genetic level. It has also been suggested that retinoids are stimulating the formation of the natural MMP inhibitors called TIMPs. Furthermore retinoids are enhancing cell renewal and differentiation. By means of those mechanisms retinoids are truly skin anti-aging ingredients. However, even their efficacy is well established, there is a demand in the cosmetic industry to further maximize their benefits.

Therefore a variety of combination has been suggested. US 2002/0 119 114 discloses combinations of lipoic acid and retinoids for treating dry skin and preventing skin aging. EP-A 610 511 discloses combinations of vitamin A and an anti-inflammatory agent. HDAC inhibitors are not described. WO 02/02074 and WO 98/13020 disclose that certain compounds enhance the conversion of retinyl esters and retinol to retinoic acid and are useful in combination with retinoids. The document is not directed to HDAC inhibitors. WO 01/74307 discloses combinations of prostaglandin derivatives with vitamin A. HDAC inhibitors are not disclosed. WO 98/36742 discloses the combination and use of Vitamin A and MMP enzyme inhibitors for the treatment of chronological aging effects on human skin, however, HDAC inhibitors are not mentioned. Retinol and derivatives thereof in combination with a certain skin lightening acid is known to be useful in the repair of photo-damaged skin or the prevention of photo-damage to skin following the exposure to UV-light, see e.g. WO 94/09756.

WO 01/08650 discloses the use of certain fatty acids together with certain retinoids. The fatty acids used are effective PPAR agonists as described in US 2003/0003068. Thus, a combination of PPAR agonists with retinoids is also useful. All combinations mentioned above are state of the art and can be further combined and improved with the present combination of a retinoid with a HDAC inhibitor described in this document.

US 6538030 discloses the use of Phenylbutyric acid and derivatives for the treatment of radiation fibrosis or skin ulcers. It also describes topical formulations. However the document does not mention combinations with retinoids and treatment of age related phenomena.

US 03/0206946 reports the use of HDAC inhibitors for the treatment of inflammation of the skin and provides a therapy for treatment of connective tissue diseases. It also provides compositions for topical treatment. It did not report a combination with a retinoid or the use to treat skin aging.

Hair loss or alopecia is a common affliction of humans. The most common form of hair loss in both males and females is patterned boldness or androgenic alopecia.

Hair follicles range in size from small, superficial, vellus follicles to large, deep, terminal follicles. The cyclic growth phases of hair follicles are telogen (resting), anagen I-III (developing), anagen IV-VI (growing) and catagen (involuting).

In the development of androgenic alopecia there is the gradual diminution of follicle size, with conversion of large, terminal follicles, producing thick, pigmented hair fibers (terminal hair) to small vellus follicles producing fine, non-pigmented hair fibers (vellus hair). In addition, the proportion of growing anagen follicles declines.

There exists a wide variety of literature regarding cosmetic preparations, in particular for cosmetic preparations for treating wrinkles and for promoting hair growth. As examples of the extensive literature it can be referred e.g. to GB 906,000, EP-A 699 429 or WO 03/086342.

WO 98/17273 reports the use of butyric acid and derivatives for the protection of hair loss. The document does not mention a necessity of a particular butyric acid derivative to be also a HDAC inhibitor neither does it mention a combination with a retinoid. Compositions, which contain butyric acid derivatives, often contain small amounts of free butyric acid or such small amounts of free butyric acid develop during storage or application of the composition. Such compositions are disadvantageous for skin and scalp treatment because of the resulting malodor.

The problem to be solved by the present application is the provision of novel compositions, in particular of cosmetic preparations for treating and preventing wrinkles and thickening of the epidermis but also preparations which are useful against other conditions which are observed with skin aging due to environmental or other external influences or due to age. The new compositions should have an activity, which is comparable to the activity of known cosmetic preparations but preferably is better than the activity of the prior art preparations.

This problem is solved on the basis of the unexpected finding that a combination of an HDAC inhibitor as defined below and a retinoid, in particular with retinal or its precursors described for example in WO 97/27836 and EP-A 391033, retinol or a retinol derivative shows synergistic activity in topical cosmetic applications for treating and preventing skin wrinkles as well as thickening and fortifying the epidermis, treatment of cellulite, but also for ameliorating the effects of aging of the skin, which may be caused by external or environmental hazards or by the natural aging of the skin.

It was found that the HDAC inhibitors have excellent effect in cosmetic applications, in particular for treating and preventing skin wrinkles, prevention and treatment of hair loss, skin lightening and prevention and treatment of age spots, as well as thickening and fortifying the epidermis, treatment of cellulite, but also for ameliorating the effects of aging of the skin, which may be caused by external or environmental hazards or by the natural aging of the skin. The present application relates to the subject matter of claims 1-10, and is generally directed to topical compositions, preferably topical cosmetic compositions, containing one or more HDAC inhibitors as defined hereinafter which contain a retinoid The retinoids show a synergistic effect with the HDAC inhibitors.

Accordingly, the present application provides a composition comprising at least one HDAC inhibitor in combination with a retinoid and a cosmetically or pharmaceutically acceptable excipient or diluent. Preferred are retinoids, which are different from retinoic acid.

HDAC inhibitors are well known in the art and described in many patents and patent applications. Known HDAC inhibitors can roughly be divided in the subclasses of non-peptide hydroxamic acids, cyclic peptides, benzamides, phenylalkyl carboxylic acids, butyric acid analogues and electrophilic ketones. Butyric acid analogues, in particular the retinoyloxy (substituted) alkylene butyrate compounds disclosed in WO 98/00127 and in particular butyric acid are preferably not present in the cosmetic preparations of the present invention.

A class of HDAC inhibitors are non-peptide hydroxamic acid inhibitors which are disclosed e.g. in WO 97/35990, US-A 5,369,108, US-A 5,608,108, US-A 5,700,811, WO 01/18171, WO 98/55449, WO 93/12075, WO 01/49290, WO 02/26696, WO 02/26703, JP 10182583, WO 99/12884, WO 01/38322, WO 01/70675 and WO 02/22577. All HDAC inhibitors disclosed in these publications are included herein by reference.

Other HDAC inhibitors are cyclic peptide inhibitors, and here it can be referred e.g. to US-A 5,620,953, US-A 5,922,837, WO 01/07042, WO 00/08048, WO 00/21979, WO 99/11659, WO 00/52033 and WO 02/0603. All HDAC inhibitors disclosed in these publications are included herein by reference.

HDAC inhibitors are also those which are based on a benzamide structure which are disclosed e.g. in Proc. Natl. Acad. Sci. USA (1999), 96: 4592-4597, but also in EP-A 847 992, US 6,174,905, JP 11269140, JP 11335375, JP 11269146, EP 974 576, WO 01/38322, WO 01/70675 and WO 01/34131. All HDAC inhibitors, which are disclosed in these documents, are included herein by reference.

Butyric acid analogues which are known HDAC inhibitors are disclosed e.g. in WO 99/37150, EP-A 1 170 008, WO 02/07722, WO 98/00127, WO 98/39965, WO 98/39966, WO 98/40066, WO 98/40065 and WO 98/40080. All HDAC inhibitors disclosed in these documents are included herein by reference. The compositions of the present invention are preferably free of butyric acid.

A further class of HDAC inhibitors are electrophilic ketone inhibitors which are disclosed e.g. in WO 01/18171 and WO 02/46129, and the HDAC inhibitors disclosed in these documents are included herein by reference.

HDAC inhibitors are also disclosed e.g. in WO 02/46144, WO 02/22577 and WO 02/30879 and the disclosure of these documents is included herein by reference.

HDAC inhibitors are also described in
- Marks, P. A., Richon, V. M., Breslow, R., Rifkind, R. A., Histone deacetylase inhibitors as new cancer drugs, Current Opinion in Oncology, 2001, 13, 477-483;
- Jung, M., Inhibitors of histone deacetylase as new anticancer agents, Current Medicinal Chemistry, 2001, 8, 1505-1511;
- Vigushin, D. M., Coombes, R. C., Histone deacetylase inhibitors in cancer treatment, Anti-Cancer Drugs, 2002, 13, 1-13;
- Remiszewski, S. W., Recent advances in the discovery of small molecule histone deacetylase inhibitors, Current Opinion in Drug Discovery & Development, 2002, 5, 487-499;
- Meinke, P. T., Liberator, P., Histone Deacetylase: A target for antiproliferative and antiprotozoal agents, Current Medicinal Chemistry, 2001, 8, 211-235;
- Marks, P. A., Rifkind, R. A., Richon, V. M., Breslow, R., Miller, T., Kelly, W. K., Histone Deacetylase and Cancer: Causes and Therapies, Nature Reviews Cancer, 2001, 1, 194-202;

Marks, P. A., Richon, V. M., Rifkind, R. A., Histone deacetylase inhibitors: inducer of differentiation or apoptosis of transformed cells, J. Nat. Cancer Inst., 2000, 92, 1210-1216
- Jung, M., Histone Deacetylases, Targets for Cancer Chemotherapy: Transcription Factors and Other Nuclear Proteins (Edt.: La Thangue, N. B., Bandara, L. B.), 2002, 123-144;
- De Ruijter, A. J. M., van Gennip, A. H., Caron, H. N., Kemp, S., van Kuilenburg, A. B. P., Histone Deacetylases (HDACs): Characterization of the classical HDAC family, Biochemical Journal, 2003, 370, 737-749;
- Kelly, W. K., O'Connor, O., Marks, P. A., Histone deacetylase inhibitors: from target to clinical trials, Expert Opin. Investig. Drugs, 2002, 11, 1695-1713.
- Curtin, M., Synthesis of novel hydroxamate and non-hydroxamate histone deacetylase inhibitors, Curr. Opinion in Drug Discovery & Development, 2004, 7(6), 848-868.
- Monneret, C., Histone Deacetylase Inhibitors, Eur. J. Med. Chem., 2005, 40, 1-13.
- Suzuki, T., Nagano, Y., Kouketru, A., Matsumura, A., Marayama, S., Kurotaki, M., Nakagawa, H., Miyata, N., Novel Inhibitors of Human, Histone Deacetylase: Design, Synthesis, Enzyme Inhibition, and cancer cell growth inhibitors of SAHA-based non-hydroxamates, J. Med. Chem., 2005, 48, 1019-1032.

The HDAC inhibitors of the above documents are also included herein by reference.

HDAC inhibitors which are included in the topical compositions of the invention have the formula 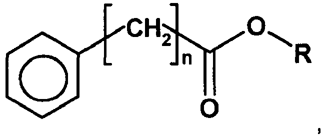 wherein R is a C₁-C₃₀ hydrocarbon, preferably C₁-C₂₀ hydrocarbon. Preferably R is C₁-C₂₀ alkyl, C₂-C₂₀ alkenyl, C₆-C₂₀ aryl, C₇-C₂₀ alkylaryl, C₇-C₂₀ arylalkyl, C₈-C₂₀ alkylarylalkyl, C₈-C₂₀ alkenylaryl, C₈-C₂₀ arylalkenyl, C₉-C₂₀ alkenylarylalkyl, C₉-C₂₀ alkylarylalkenyl, C₁₀-C₂₀ alkenylarylalkenyl, and each of the above preferred residues can be unsubstituted or substituted by C₁-C₆ alkyl or C₂-C₆ alkenyl,
n is an integer of 1 to 5.

HDAC inhibitors which are used in combination with a retinoid for providing a cosmetic effect selected from treating and preventing skin wrinkles, ameliorating the effects of aging of the skin and thickening of the epidermis have the formula 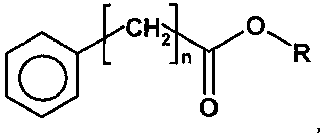 wherein R is hydrogen, or C₁-C₃₀ hydrocarbon, preferably C₁-C₂₀ hydrocarbon. Preferably R is hydrogen, C₁-C₂₀ alkyl, C₂-C₂₀ alkenyl, C₆-C₂₀ aryl, C₇-C₂₀ alkylaryl, C₇-C₂₀ arylalkyl, C₈-C₂₀ alkylarylalkyl, C₈-C₂₀ alkenylaryl, C₈-C₂₀ arylalkenyl, C₉-C₂₀ alkenylarylalkyl, C₉-C₂₀ alkylarylalkenyl, C₁₀-C₂₀ alkenylarylalkenyl, and each of the above preferred residues can be unsubstituted or substituted by C₁-C₆ alkyl or C₂-C₆ alkenyl,
n is an integer of 1 to 5.

R can be retinyl but preferably is not retinyl.

R is particularly preferred C₁-C₂₀ alkyl or C₂-C₂₀ alkenyl.

In the context of the present specification an "alkenyl" group can comprise 1 or more, preferably 1 to 4 or 1 to 3 double bonds. Alkyl and alkenyl groups can be straight chained, branched or cyclic or comprise cyclic elements.

Phenylbutyryl carboxylic acid derivatives should be particularly mentioned. The acid and acid derivatives can be used for providing a cosmetic effect as defined above in the free acid form. Phenylacetyl derivatives (n = 1) are not preferred due to the unpleasant smell of the free acid. The free acid can be an impurity in an ester, or will form upon formulation, storage of the formulation or during application of the ester-compounds.

HDAC inhibitors are also described by Jung et al., in J. Med. Chem. 2002, 45, 3296-3309 and in Bioorg. Med. Chem. Lett. 1997, 7, 1655-1658. Particularly mentioned are molecules having the following structure:

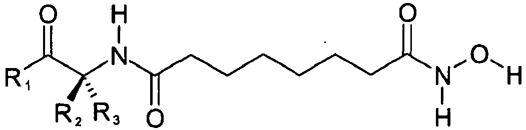

With R₁ being -OEt, -NH₂, or -OH and R₂ being -H, -NHCH₂Ph, -CH(CH₃)₂ and R₃ being H or -NHCH₂Ph.

Furthermore HDAC inhibitors described by Chen et al. in J. Med. Chem. 2004, 47, 467-474 should be mentioned. Particularly mentioned are molecules, which have the formula: 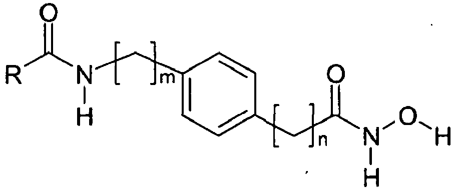 with R being -CH₂CH₂CH₃, -CH₂Ph, -CH₂(CH₂)₂Ph and m being 0 or 1 and n being 0. Not preferred are all derivatives of Valproic acid.

Another HDAC inhibitor is Trichostatin A.

Furthermore, HDAC inhibitors described by Jung et al. in J. Med. Chem. 1999, 42, 4669-4679 having the following formula are mentioned:

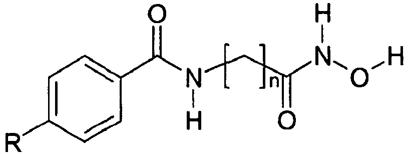

Particular suitable are molecules with R = OMe or H and n = 5.

Furthermore, HDAC inhibitors described in WO 04/009536 and WO 02/076941 are mentioned. Especially molecules having the following formula 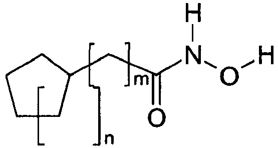 are mentioned. Cyclohexane derivatives having m = 1 - 3 and n = 2 are HDAC inhibitors.

Furthermore, HDAC inhibitors described by Marks et al. in Proc. Nat. Acad. Sci. USA 1991, 88, 5542-5546 having the following formula are mentioned:

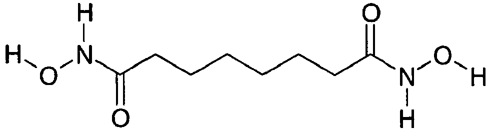

The other active ingredient which is present in the compositions of the invention and which surprisingly synergistically increases the cosmetic activity of the HDAC inhibitors is a retinoid, in particular retinal, retinol or a derivative thereof, such as an acetal or an imine of retinal. Retinol is also known as vitamin A₁ and has the following structure.

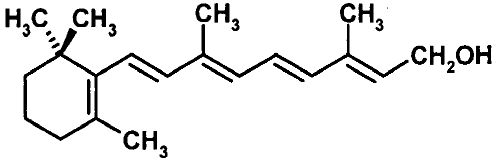

Derivatives of retinol which can be used according to the invention are known and preferred derivatives are β-carotin, 3,4-didehydroretinol (vitamin A₂) and the stereoisomers of retinol, in particular the 9-cis and the 13-cis retinol. Preferred derivatives of retinol are also esters of retinol of the formula 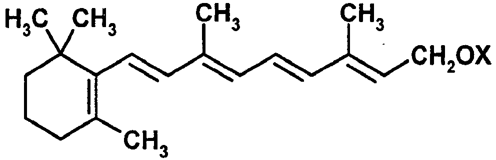 where X represents COR where R represents a group chosen from branched or unbranched, alkyl, aryl, alkylaryl or alkenyl groups having an average of from 1 to 20, preferably from 1 to 10, and particularly preferred is also an average of about 15 (palmityl residue) carbon atoms. The above formulas should be read to include all stereoisomers of the retinol derivatives, in particular those compounds in which one or more C=C double bonds are cis.

Preferred retinoids have the general formula 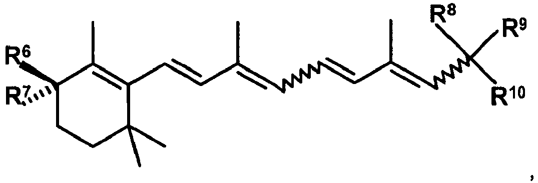
wherein R⁶ and R⁷ are independently of each other hydrogen or hydroxy or R⁶ and R⁷ together form an oxygen atom
R⁸ and R⁹ are hydrogen or R⁸ and R⁹ together form an oxygen atom
R¹⁰ is hydrogen, hydroxy or a residue OR¹¹, provided that if R⁸ and R⁹ together form oxygen R¹⁰ is not hydroxy.
R¹¹ is C₁-C₂₀ alkyl (preferably C₁-C₆ alkyl), C₂-C₂₀ alkenyl (preferably C₂-C₆ alkenyl) or C₇-C₁₀ alkylaryl (preferably phenyl-(C₁-C₄)-alkyl) a residue -C(O)-R¹²,
R¹² is C₁-C₂₀ alkyl (preferably C₁-C₆ alkyl) or C₂-C₂₀ alkenyl (preferably C₂-C₆ alkenyl).

The double bond in position 9 is cis or trans, and the double bond in position 13 is cis or trans. Most preferred are the all-trans compounds.

Examples of preferred retinol derivatives include:
retinyl acetate
retinyl phenylbutyrate
retinyl propionate
retinyl octanoate
retinyl laurate
retinyl palmitate
retinyl oleate
retinyl linoleate
retinyl alkyl carbonate
retinoxytrimethylsilane

all trans-retinal

methoxy PEG-12 retinamide.

Preferably the composition comprises retinol, most preferably the composition comprises the trans-isomer of retinol.

The term retinoid also encompasses retinoic acid, but retinoic acid is not preferred according to the invention.

The compositions of the present invention are preferably cosmetic compositions or cosmetic preparations, but they can also be pharmaceutical compositions.

The term "cosmetic preparation" or "cosmetic composition" as used in the present application refers to cosmetic compositions as defined under the heading "Kosmetika" in Römpp Lexikon Chemie, 10th edition 1997, Georg Thieme Verlag Stuttgart, New York.

The compositions of the present invention contain the HDAC inhibitor and a retinoid together with cosmetically or pharmaceutically acceptable excipients or diluents. If nothing else is stated, the excipients, additives, diluents, etc. mentioned in the following are suitable for both pharmaceutical and cosmetic compositions.

If nothing else is stated, in this application parts and percentages are per weight and are based on the weight of the composition.

The compositions of the present invention are topical compositions, such as liquid or solid oil-in-water emulsions, water-in-oil emulsions, multiple emulsions, microemulsions, PET-emulsions, bickering emulsions, hydrogels, alcoholic gels, lipogels, one or multiphase solutions, foams, ointments, plasters, suspensions, powders, crèmes, cleanser, soaps and other usual compositions, which can also be applied by pens, as masks or as sprays.

The compositions of the invention can also contain usual cosmetic or pharmaceutical adjuvants and additives, such as preservatives/ antioxidants, fatty substances/ oils, water, organic solvents, silicones, thickeners, softeners, emulsifiers, sunscreens, antifoaming agents, moisturizers, fragrances, surfactants, fillers, sequestering agents, anionic, cationic, nonionic or amphoteric polymers or mixtures thereof, propellants, acidifying or basifying agents, dyes, colorants, pigments or nanopigments, e.g. those suited for providing a photoprotective effect by physically blocking out ultraviolet radiation, or any other ingredients usually formulated into cosmetics or medicaments.

An additional amount of antioxidants/ preservatives is generally preferred. Based on the invention all known antioxidants usually formulated into cosmetics or medicaments can be used. Especially preferred are antioxidants chosen from the group consisting of amino acids (e.g. glycine, histidine, tyrosine, tryptophan) and their derivatives, imidazole (e.g. urocanic acid) and derivatives, peptides such as D,L-carnosine, D-carnosine, L-carnosine and derivatives (e.g. anserine), carotenoids, carotenes (e.g. α-carotene, β-carotene, lycopene) and derivatives, chlorogenic acid and derivatives, lipoic acid and derivatives (e.g. dihydrolipoic acid), aurothioglucose, propylthiouracil and other thiols (e.g. thioredoxine, glutathione, cysteine, cystine, cystamine and its glycosyl-, N-acetyl-, methyl-, ethyl-, propyl-, amyl-, butyl- and lauryl-, palmitoyl-, oleyl-, γ-linoleyl-, cholesteryl-and glycerylester) and the salts thereof, dilaurylthiodipropionate, distearylthiodipropionate, thiodipropionic acid and its derivatives (ester, ether, peptides, lipids, nucleotides, nucleosides and salts) as well as sulfoximine compounds (such as buthioninsulfoximine, homocysteinsulfoximine, buthioninsulfone, penta-, hexa-, heptathioninsulfoximine) in very low compatible doses (e.g. pmol to µmol/kg), additionally (metal)-chelators (such as α-hydroxyfatty acids, palmic-, phytinic acid, lactoferrin), α-hydroxyacids (such as citric acid, lactic acid, malic acid), huminic acid, gallic acid, gallic extracts, bilirubin, biliverdin, EDTA, EGTA and its derivatives, unsaturated fatty acids and their derivatives (such as γ-linoleic acid, linolic acid, oleic acid), folic acid and its derivatives, ubiquinone and ubiquinol and their derivatives, vitamin C and derivatives (such as ascorbylpalmitate and ascorbyltetraisopalmitate, Mg-ascorbylphosphate, Na-ascorbylphosphate, Na-ascorbylacetate), tocopherol and derivatives (such as vitamin-E-acetate), mixtures of nat. vitamin E, vitamin A and derivatives (vitamin-A-palmitate and -acetate) as well as coniferylbenzoate, rutinic acid and derivatives, α-glycosylrutin, ferulic acid, furfurylidenglucitol, carnosine, butylhydroxytoluene, butylhydroxyanisole, trihydroxybutyrophenone, urea and its derivatives, mannose and derivatives, zinc and derivatives (e.g. ZnO, ZnSO₄), selenium and derivatives (e.g. selenomethionin), stilbenes and derivatives (such as stilbenoxide, trans-stilbenoxide) and suitable derivatives (salts, esters, ethers, sugars, nucleotides, nucleosides, peptides and lipids) of the named active ingredients. One or more preservatives/antioxidants may be present in an amount about 0.01 wt.% to about 10 wt.% of the total weight of the composition of the present invention. Preferably, one or more preservatives/antioxidants are present in an amount about 0.1 wt.% to about 1 wt.%.

Typically topical formulations also contain surface active ingredients like emulsifiers, solubilizers and the like. An emulsifier enables two or more immiscible components to be combined homogeneously. Moreover, the emulsifier acts to stabilize the composition. Emulsifiers that may be used in the present invention in order to form O/W, W/O, O/W/O or W/O/W emulsions/ microemulsions include sorbitan oleate, sorbitan sesquioleate, sorbitan isostearate, sorbitan trioleate, polyglyceryl-3-diisostearate, polyglycerol esters of oleic/isostearic acid, polyglyceryl-6 hexaricinolate, polyglyceryl-4-oleate, polyglyceryl-4 oleate/PEG-8 propylene glycol cocoate, oleamide DEA, TEA myristate, TEA stearate, magnesium stearate, sodium stearate, potassium laurate, potassium ricinoleate, sodium cocoate, sodium tallowate, potassium castorate, sodium oleate, and mixtures thereof. Further suitable emulsifiers are phosphate esters and the salts thereof such as cetyl phosphate (Amphisol^{®} A), diethanolamine cetyl phosphate (Amphisol^{®}), potassium cetyl phosphate (Amphisol^{®} K), sodium glyceryl oleate phosphate, hydrogenated vegetable glyceride phosphates and mixtures thereof. Furthermore, one or more synthetic polymers may be used as an emulsifier. For example, PVP eicosene copolymer, acrylates/C₁₀₋₃₀ alkyl acrylate crosspolymer, acrylates/steareth-20 methacrylate copolymer, PEG-22/dodecyl glycol copolymer, PEG-45/dodecyl glycol copolymer, and mixtures thereof. The preferred emulsifiers are cetyl phosphate (Amphisol^{®} A), diethanolamine cetyl phosphate (Amphisol^{®}), potassium cetyl phosphate (Amphisol^{®} K), PVP Eicosene copolymer, acrylates/C₁₀₋₃₀-alkyl acrylate crosspolymer, PEG-20 sorbitan isostearate, sorbitan isostearate, and mixtures thereof. The one or more emulsifiers are present in a total amount about 0.01 wt.% to about 20 wt.% of the total weight of the composition of the present invention. Preferably, about 0.1 wt.% to about 10 wt.% of emulsifiers is used.

The lipid phase of the topical compositions can advantageously be chosen from:
mineral oils and mineral waxes;
oils such as triglycerides of caprinic acid or caprylic acid and castor oil;
oils or waxes and other natural or synthetic oils, in a preferred embodiment esters of fatty acids with alcohols e.g. isopropanol, propylene glycol, glycerin or esters of fatty alcohols with carboxylic acids or fatty acids;
alkylbenzoates; and/or
silicone oils such as dimethylpolysiloxane, diethylpolysiloxane, diphenylpolysiloxane, cyclomethicones and mixtures thereof.

Exemplary fatty substances which can be incorporated in the oil phase of the emulsion, microemulsion, oleo gel, hydrodispersion or lipodispersion of the present invention are advantageously chosen from esters of saturated and/or unsaturated, linear or branched alkyl carboxylic acids with 3 to 30 carbon atoms, and saturated and/or unsaturated, linear and/or branched alcohols with 3 to 30 carbon atoms as well as esters of aromatic carboxylic acids and of saturated and/or unsaturated, linear or branched alcohols of 3-30 carbon atoms. Such esters can advantageously be selected from octylpalmitate, octylcocoate, octylisostearate, octyldodecylmyristate, cetearylisononanoate, isopropylmyristate, isopropylpalmitate, isopropylstearate, isopropyloleate, n-butylstearate, n-hexyllaureate, n-decyloleat, isooctylstearate, isononylstearate, isononylisononanoate, 2-ethyl hexylpalmitate, 2-ethylhexyllaurate, 2-hexyldecylstearate, 2-octyldodecylpalmitate, stearylheptanoate, oleyloleate, oleylerucate, erucyloleate, erucylerucate, tridecylstearate, tridecyltrimellitate, as well as synthetic, half-synthetic or natural mixtures of such esters e.g. jojoba oil.

Other fatty components suitable for use in the topical compositions of the present invention include polar oils such as lecithins and fatty acid triglycerides, namely triglycerol esters of saturated and/or unsaturated, straight or branched carboxylic acid with 8 to 24 carbon atoms, preferably of 12 to 18 carbon-atoms whereas the fatty acid triglycerides are preferably chosen from synthetic, half synthetic or natural oils (e.g. cocoglyceride, olive oil, sun flower oil, soybean oil, peanut oil, rape seed oil, sweet almond oil, palm oil, coconut oil, castor oil, hydrogenated castor oil, wheat oil, grape seed oil, macadamia nut oil and others); apolar oils such as linear and/ or branched hydrocarbons and waxes e.g. mineral oils, vaseline (petrolatum); paraffins, squalane and squalene, polyolefins, hydrogenated polyisobutenes and isohexadecanes, favored polyolefins are polydecenes; dialkyl ethers such as dicaprylylether; linear or cyclic silicone oils such as preferably cyclomethicones (octamethylcyclotetrasiloxane; cetyldimethicone, hexamethylcyclotrisiloxane, polydimethylsiloxane, poly(methylphenylsiloxane) and mixtures thereof.

Other fatty components which can advantageously be incorporated in topical compositions of the present invention are isoeikosane; neopentylglycoldiheptanoate; propyleneglycoldicaprylate/ dicaprate; caprylic/ capric/ diglycerylsuccinate; butyleneglycol caprylat/caprat; C₁₂₋₁₃-alkyllactate; di-C₁₂₋₁₃ alkyltartrate; triisostearin; dipentaerythrityl hexacaprylat/hexacaprate; propylenglycolmonoisostearate; tricaprylin; dimethylisosorbid. Especially beneficial is the use of mixtures C₁₂₋₁₅-alkylbenzoate and 2-ethylhexylisostearate, mixtures C₁₂₋₁₅-alkylbenzoate and isotridecylisononanoate as well as mixtures of C₁₂₋₁₅-alkylbenzoate, 2-ethylhexylisostearate and isotridecylisononanoate. The oily phase of the compositions of the present invention can also contain natural vegetable or animal waxes such as bee wax, china wax, bumblebee wax and other waxes of insects as well as shea butter and cocoa butter.

A moisturizing agent may be incorporated into a topical composition of the present invention to maintain hydration or rehydrate the skin. Moisturizers that prevent water from evaporating from the skin by providing a protective coating are called emollients. Additionally an emollient provides a softening or soothing effect on the skin surface and is generally considered safe for topical use. Preferred emollients include mineral oils, lanolin, petrolatum, capric/caprylic triglyceraldehydes, cholesterol, silicones such as dimeticone, cyclometicone, almond oil, jojoba oil, avocado oil, castor oil, sesame oil, sunflower oil, coconut oil and grape seed oil, cocoa butter, olive oil aloe extracts, fatty acids such as oleic and stearic, fatty alcohols such as cetyl and hexadecyl (ENJAY), diisopropyl adipate, hydroxybenzoate esters, benzoic acid esters of C₉₋₁₅-alcohols, isononyl iso-nonanoate, ethers such as polyoxypropylene butyl ethers and polyoxypropylene cetyl ethers, and C₁₂₋₁₅- alkyl benzoates, and mixtures thereof. The most preferred emollients are hydroxybenzoate esters, aloe vera, C₁₂₋₁₅-alkyl benzoates, and mixtures thereof. An emollient is present in an amount of about 1 wt.% to about 20 wt.% of the total weight of the composition. The preferred amount of emollient is about 2 wt.% to about 15 wt.%, and most preferably about 4 wt.% to about 10 wt.%.

Moisturizers that bind water, thereby retaining it on the skin surface are called humectants. Suitable humectants can be incorporated into a topical composition of the present invention such as glycerin, polypropylene glycol, polyethylene glycol, lactic acid, pyrrolidone carboxylic acid, urea, phospholipids, collagen, elastin, ceramides, lecithin sorbitol, PEG-4, and mixtures thereof. Additional suitable moisturizers are polymeric moisturizers of the family of water soluble and/ or swellable/ and/ or with water gelating polysaccharides such as hyaluronic acid, chitosan and/or a fucose rich polysaccharide which is e.g. available as Fucogel®1000 (CAS-Nr. 178463-23-5) by SOLABIA S. One or more humectants are optionally present at about 0.5 wt.% to about 8 wt.% in a composition of the present invention, preferably about 1 wt.% to about 5 wt.%.

The aqueous phase of the preferred topical compositions of the present invention can contain the usual cosmetic or pharmaceutical additives such as alcohols, especially lower alcohols, preferably ethanol and/ or isopropanol, low diols or polyols and their ethers, preferably propyleneglycol, glycerin, ethyleneglycol, ethyleneglycol monoethyl- or monobutylether, propyleneglycol monomethyl- or -monoethyl- or-monobutylether, diethyleneglycol monomethyl-or monoethylether and analogue products, polymers, foam stabilizers; electrolytes and especially one or more thickeners. Thickeners that may be used in formulations of the present invention to assist in making the consistency of a product suitable include carbomer, siliciumdioxide, magnesium and/ or aluminium silicates, beeswax, stearic acid, stearyl alcohol polysaccharides and their derivatives such as xanthan gum, hydroxypropyl cellulose, polyacrylamides, acrylate crosspolymers preferably a carbomer, such as carbopole^{®} of type 980, 981, 1382, 2984, 5984 alone or mixtures thereof. Suitable neutralizing agents which may be included in the composition of the present invention to neutralize components such as e.g. an emulsifier or a foam builder/stabilizer include but are not limited to alkali hydroxides such as a sodium and potassium hydroxide; organic bases such as diethanolamine (DEA), triethanolamine (TEA), aminomethyl propanol, and mixtures thereof; amino acids such as arginin and lysine and any combination of any foregoing. The neutralizing agent can be present in an amount of about 0.01 wt.% to about 8 wt.% in the composition of the present invention, preferably, 1 wt.% to about 5 wt.%.

The addition of electrolytes into the composition of the present invention may be necessary to change the behavior of a hydrophobic emulsifier. Thus, the emulsions/ microemulsions of this invention may contain preferably electrolytes of one or several salts including anions such as chloride, sulfates, carbonate, borate and aluminate, without being limited thereto. Other suitable electrolytes can be on the basis of organic anions such as, but not limited to, lactate, acetate, benzoate, propionate, tartrate and citrate. As cations preferably ammonium, alkylammonium, alkali- or alkaline earth metals, magnesium-, iron- or zinc-ions are selected. Especially preferred salts are potassium and sodium chloride, magnesium sulfate, zinc sulfate and mixtures thereof. Electrolytes can be present in an amount of about 0.01 wt.% to about 8 wt.% in the composition of the present invention.

The topical compositions of the invention can preferably be provided in the form of a lotion, a thickened lotion, a gel, a cream, a milk, an ointment, a powder or a solid tube stick and can be optionally be packaged as an aerosol and can be provided in the form of a mousse, foam or a spray. The compositions according to the invention can also be in the form of a suspension or dispersion in solvents or fatty substances, or alternatively in the form of an emulsion or microemulsion (in particular of O/W or W/O type, O/W/O or W/O/W-type), such as a cream or a milk, a vesicular dispersion, in the form of an ointment, a gel, a solid tube stick or an aerosol mousse. The emulsions can also contain anionic, nonionic, cationic or amphoteric surfactants.

The topical application is preferably at least once per day, e.g. twice or triple times a day. Usually it takes at least two weeks until the desired effect is achieved. However, it can take several weeks or even months until the desired effect is fully maximized.

The amount of the topical composition, which is to be applied to the skin depends on the concentration of the active ingredients in the compositions and the desired cosmetic or pharmaceutical effect. For example, application can be such that a crème is applied to the skin. A crème is usually applied in an amount of 2 mg creme/cm² skin. The amount of the composition which is applied to the skin is, however, not critical, and if with a certain amount of applied composition the desired effect cannot be achieved, a higher concentration of the active ingredients can be used e.g. by applying more of the composition or by applying compositions which contain more active ingredient.

According to the invention for preparing the compositions the active ingredients can be used as such or in an encapsulated form, for example in a liposomal form. Liposomes are preferably formed with lecithins with or without addition of sterols or phytosterols. The encapsulation of the active ingredients can be alone or together with other active ingredients. It is possible to encapsulate only the HDAC inhibitors or only the retinol or the derivative thereof, but it is also possible to encapsulate both the HDAC inhibitor or the retinol or derivative thereof, either together or in separate capsules.

Other embodiments include solid or semisolid capsules aiming to protect the retinoid from degradation or for controlled delivery. The capsule may contain the retinoid alone or together with the HDAC inhibitor. Suitable encapsulation technologies are for example described in WO 0180823, WO 9903450, WO 9317784 or in Fragrance Journal (2001), 29(2), 83-90.

Also suitable is a combination of two or more HDAC inhibitors with a retinoid or a combination of two or more retinoids.

In the composition of the invention the HDAC inhibitor and the retinol or the derivative thereof are independently contained in an amount of preferably 0.0001 wt.-% to about 50 wt.-%, based on the total weight of the composition. More preferably, the HDAD inhibitor and the retinol or derivative thereof are independently contained in the composition in an amount of about 0.01 wt.-% to about 20 wt.-%, more preferably in an amount of about 0.01 wt.-% to about 1 wt.-%, in particular in an amount of about 0.1 wt.-%, based on the total amount of the composition.

Regarding the kind of the topical preparation and the preparation of the topical preparations as well as for further suitable additives, it can be referred to the pertinent literature, e.g. to Novak G.A., Die kosmetischen Präparate - Band 2, Die kosmetischen Präparate - Rezeptur, Rohstoffe, wissenschaftliche Grundlagen (Verlag für Chem. Industrie H. Ziolkowski KG, Augsburg).

Additionally the composition of the present invention may contain UV-A and UV-B filters. Examples of UV-B or broad spectrum screening agents, i.e. substances having absorption maximums between about 290 and 340 nm, which are preferred for combination with the compounds of the present invention, are the following organic and inorganic compounds:
Acrylates such as 2-ethylhexyl 2-cyano-3,3-diphenylacrylate (octocrylene, PARSOL® 340), ethyl 2-cyano-3,3-diphenylacrylate and the like;
   --- Camphor derivatives such as 4-methyl benzylidene camphor (PARSOL® 5000), 3-benzylidene camphor, camphor benzalkonium methosulfate, polyacrylamidomethyl benzylidene camphor, sulfo benzylidene camphor, sulphomethyl benzylidene camphor, therephthalidene dicamphor sulfonic acid and the like;
   --- Cinnamate derivatives such as octyl methoxycinnamate (PARSOL® MCX), ethoxyethyl methoxycinnamate, diethanolamine methoxycinnamate (PARSOL® Hydro), isoamyl methoxycinnamate and the like as well as cinnamic acid derivatives bond to siloxanes;
   --- p-aminobenzoic acid derivatives, such as p-aminobenzoic acid, 2-ethylhexyl p-dimethylaminobenzoate, N-oxypropylenated ethyl p-aminobenzoate, glyceryl p-aminobenzoate,
   --- Benzophenones such as benzophenone-3, benzophenone-4, 2,2', 4, 4'-tetrahydroxy-benzophenone, 2,2'-dihydroxy-4,4'-dimethoxybenzophenone and the like;
   --- Esters of Benzalmalonic acid such as di-(2-ethylhexyl) 4-methoxybenzalmalonate;
   --- Esters of 2-(4-ethoxy-anilinomethylene)propandioic acid such as 2-(4-ethoxy anilinomethylene)propandioic acid diethyl ester as described in the European Patent Publication EP 0895 776;
   --- Organosiloxane compounds containing benzmalonate groups as described in the European Patent Publications EP 0358584 B1, EP 0538431 B1 and EP 0709080 A1;
   --- Drometrizole trisiloxane (Mexoryl XL);
   --- Pigments such as microparticulated TiO₂, and the like. The term "microparticulated" refers to a particle size from about 5 nm to about 200 nm, particularly from about 15 nm to about 100 nm. The TiO₂ particles may also be coated by metal oxides such as e.g. aluminium or zirconium oxides or by organic coatings such as e.g. polyols, methicone, aluminium stearate, alkyl silane. Such coatings are well known in the art.
   --- Imidazole derivatives such as e.g. 2-phenyl benzimidazole sulfonic acid and its salts (PARSOL®HS). Salts of 2-phenyl benzimidazole sulfonic acid are e.g. alkali salts such as sodium- or potassium salts, ammonium salts, morpholine salts, salts of primary, sec. and tert. amines like monoethanolamine salts, diethanolamine salts and the like.
   --- Salicylate derivatives such as isopropylbenzyl salicylate, benzyl salicylate, butyl salicylate, octyl salicylate (NEO HELIOPAN OS), isooctyl salicylate or homomenthyl salicylate (homosalate, HELIOPAN) and the like.
   --- Triazine derivatives such as octyl triazone (UVINUL T-150), dioctyl butamido triazone (UVASORB HEB), bis ethoxyphenol methoxyphenyl triazine (Tinosorb S) and the like.
   --- Encapsulated UV-filters such as encapsulated octyl methoxy cinnamate (Eusolex UV-pearls) and the like.

Examples of broad spectrum or UV A screening agents i.e. substances having absorption maximums between about 320 and 400 nm, which are preferred for combination with the compounds of the present invention are the following organic and inorganic compounds:
--- Dibenzoylmethane derivatives such as 4-tert. butyl-4'-methoxydibenzoylmethane (PARSOL® 1789), dimethoxydibenzoylmethane, isopropyldibenzoylmethane and the like;
--- Benzotriazole derivatives such as 2,2'-methylene-bis-(6-(2H-benzotriazole-2-yl)-4-(1,1,3,3,-tetramethylbutyl)-phenol (TINOSORB M) and the like;
--- phenylene-1,4-bis-benzimidazolsulfonic acids or salts such as 2,2-(1,4-phenylene)bis-(1H-benzimidazol-4,6-disulfonic acid) (Neoheliopan AP);
--- amino substituted hydroxybenzophenones such as 2-(4-Diethylamino-2-hydroxy-benzoyl)-benzoic acid hexylester as described in the European Patent Publication EP 1046391;
--- Pigments such as microparticulated ZnO or TiO₂ and the like. The term "microparticulated" refers to a particle size from about 5 nm to about 200 nm, particularly from about 15 nm to about 100 nm. The particles may also be coated by other metal oxides such as e.g. aluminium or zirconium oxides or by organic coatings such as e.g. polyols, methicone, aluminium stearate, alkyl silane. Such coatings are well known in the art.

As dibenzoylmethane derivatives have limited photostability it may be desirable to photostabilize these UV-A screening agents. Thus, the term "conventional UV-A screening agent" also refers to dibenzoylmethane derivatives such as e.g. PARSOL® 1789 stabilized by, e.g.,
--- 3,3-Diphenylacrylate derivatives as described in the European Patent Publications EP-A 0 514 491 and EP-A 0 780 119;
--- Benzylidene camphor derivatives as described in the US Patent No. 5,605,680;
--- Organosiloxanes containing benzmalonate groups as described in the European Patent Publications EP-A 0358584, EP-A 0538431 and EP-A 0709080.

A good overview of UV-A- and UV-B-filters which can be added to the compositions of the present invention can also be found in DE-A 103 27 432. All UV-filter compounds disclosed in this document are also useful as components for the compositions of the present invention and are included herein by reference.

The composition of the present invention can also contain additional pharmaceutically or cosmetically active ingredients, in particular for preventing or reducing acne, wrinkles, lines, atrophy, inflammation, as well as topical anesthetics, artificial tanning agents and accelerators, antimicrobial agents, and antifungal agents, and sunscreening actives.

Examples are peptides (e.g., Matrixyl^{™} [pentapeptide derivative]), farnesol, bisabolol, phytantriol, glycerol, urea, guanidine (e.g., amino guanidine); vitamins and derivatives thereof such as ascorbic acid, vitamin A (e.g., retinoid derivatives such as retinyl palmitate or retinyl propionate), vitamin E (e.g., tocopherol acetate), vitamin B₃ (e.g., niacinamide) and vitamin B₅ (e.g., octyl palmitate and tribehenin and sorbitan isostearate and palmitoyl-oligopeptide), anti-acne medicaments (resorcinol, salicylic acid, and the like); antioxidants (e.g., phytosterols, lipoic acid); flavonoids (e.g., isoflavones, phytoestrogens); skin soothing and healing agents such as aloe vera extract, allantoin and the like; chelators and sequestrants; and agents suitable for aesthetic purposes such as essential oils, fragrances, skin sensates, opacifiers, aromatic compounds (e.g., clove oil, menthol, camphor, eucalyptus oil, and eugenol), desquamatory actives, anti-acne actives, vitamin B₃ compounds, anti-oxidants, peptides, hydroxy acids, radical scavengers, chelators, farnesol, anti-inflammatory agents, topical anesthetics, tanning actives, skin lightening agents, anti-cellulite agents, flavonoids, antimicrobial actives, and antifungal actives, in particular bisabolol, alkyldiols such as 1,2-pentandiol, hexandiol or 1,2-octandiol, vitamins, panthenol, phytol, phytantriol, ceramide and pseudoceramides, amino acids and bioactive peptides, protein hydrolysates, AHA acids, polyunsaturated fatty acids, plant extracts, DNA or RNA and their fragmentation products, carbohydrates.

Preferred additional active ingredients are also Biotin, lipoic acid, conjugated fatty acids, Carnitin, Acyl-Carnitin, Vit. E , Vit A, Vit C, B3, B6, B12, Panthenol, K1, Phytantriol, Oligopeptides, Carnosin, Biochinonen, Phytofluen, Phytoen, folic acid and their corresponding derivatives.

The term "composition" as used in the present invention also encompasses an embodiment wherein the composition is present in two separate parts, wherein one part contains the HDAC inhibitor and the other part contains the retinoid. Regarding the preparation, the ingredients, the contents and dosages of the separate parts of the composition, it can be referred to the above disclosure regarding the compositions of the present invention which contain both the HDAC inhibitor and the retinoid in admixture.

The ratio of the HDAC inhibitor to the retinoid is preferably 1000:1 to 1:1000, more preferably about 100:1 to 1:100 and in particular 30:1 to 1 :30.

The HDAC inhibitors or the retinoid can also be present as hydrates or solvates, and the hydrates and solvates of the active ingredients are also encompassed by the present invention.

The following examples exemplify the invention, but they should not be construed as limiting the invention.

**Example 1 to 8 Anti-aging formulations (examples 2 to 8 are reference examples)**

| **Example formulation number Ingredients** | **1 % (w/w)** | **2 % (w/w)** | **3 % (w/w)** | **4 % (w/w)** | **5 % (w/w)** | **6 % (w/w)** | **7 % (w/w)** | **8 % (w/w)** |
|---|---|---|---|---|---|---|---|---|
| Glyceryl Myristate | 4.00 | 4.00 | 4.00 | 4.00 | 4.00 | 4.00 | 4.00 | 4.00 |
| Cetyl Alcohol | 2.00 | 2.00 | 2.00 | 2.00 | 2.00 | 2.00 | 2.00 | 2.00 |
| Steareth-2 | 2.00 | 2.00 | 2.00 | 2.00 | 2.00 | 2.00 | 2.00 | 2.00 |
| Steareth-21 | 2.00 | 2.00 | 2.00 | 2.00 | 2.00 | 2.00 | 2.00 | 2.00 |
| Isopropyl Myristate | 5.00 | 5.00 | 5.00 | 5.00 | 5.00 | 5.00 | 5.00 | 5.00 |
| Tocopheryl Acetate | 0.50 | 0.50 | 0.50 | 0.50 | 0.50 | 0.50 | 0.50 | 0.50 |
| Ethylhexyl Methoxycinnamate | 4.00 | 4.00 | 4.00 | 4.00 | 4.00 | 4.00 | 4.00 | 4.00 |
| Ethylhexyl Salicylate | 2.00 | 2.00 | 2.00 | 2.00 | 2.00 | 2.00 | 2.00 | 2.00 |
| Butyl Methoxydibenzoylmethane | 1.00 | 1.00 | 1.00 | 1.00 | 1.00 | 1.00 | 1.00 | 1.00 |
| Trichostatin A | - | 0.01 | - | - | - | - | - | - |
| Almond Oil | 2.00 | 2.00 | 2.00 | 2.00 | 2.00 | 2.00 | 2.00 | 2.00 |
| BHT | 0.05 | 0.05 | 0.05 | 0.05 | 0.05 | 0.05 | 0.05 | 0.05 |
| Phenoxyethanol & Methylparaben & Ethylparaben & Propylparaben & Butylparaben & Isopropylparaben | 0.80 | 0.80 | 0.80 | 0.80 | 0.80 | 0.80 | 0.80 | 0.80 |
| Water | Ad.100 | Ad. 100 | Ad.100 | Ad.100 | Ad.100 | Ad.100 | Ad.100 | Ad. 100 |
| Disodium EDTA | 0.10 | 0.10 | 0.10 | 0.10 | 0.10 | 0.10 | 0.10 | 0.10 |
| D-Panthenol | 0.30 | 0.30 | 0.30 | 0.30 | 0.30 | 0.30 | 0.30 | 0.30 |
| Propylene Glycol | 4.00 | 4.00 | 4.00 | 4.00 | 4.00 | 4.00 | 4.00 | 4.00 |
| Polyacrylamide & C13-14 Isoparaffin & Laureth-7 | 0.50 | 0.50 | 0.50 | 0.50 | 0.50 | 0.50 | 0.50 | 0.50 |
| Phenylbutyric Acid | 0.30 | - | - | - | - | - | - | - |
| 4-Phenylbutyric Hydroxamic Acid | - | - | 0.20 | - | - | - | - | - |
| 3-Cyclohexyl-N-hydroxy-propionamide | - | - | - | 0.10 | - | - | - | - |
| 2-Cyclohexyl-N-hydroxy-acetamide | - | - | - | - | 0.10 | - | - | - |
| N-(5-Hydroxycarbamoyl-pentyl)-4-methoxy-benzamide | - | - | - | - | - | 0.10 | - | - |
| N-Hydroxy-4-[(4-phenyl-butyrylamino)-methyl]-benzamide | - | - | - | - | - | - | 0.10 | - |
| Octanedioic acid bis-hydroxyamide | - | - | - | - | - | - | - | 0.10 |
| Retinol 15D (Caprylic/Capric Triglyceride & Retinol) | 0.50 | 0.50 | - | - | 0.50 | 0.50 | 0.50 | 0.50 |
| Retinol Acetate 2.8 MI | - | - | 0.10 | 0.10 | - | - | - | - |
| Triethanolamine | q.s. | q.s. | q.s. | q.s. | q.s. | q.s. | q.s. | q.s. |

**Examples 9 -16 facial treatment formulations (examples 10 to 16 are reference examples)**

| **Formulation No. Ingredients** | **9 % (wlw)** | **10 % (w/w)** | **11 % (w/w)** | **12 % (w/w)** | **13 % (wlw)** | **14 % (w/w)** | **15 % (wlw)** | **16 % (w/w)** |
|---|---|---|---|---|---|---|---|---|
| Glyceryl Myristate | 5.00 | 5.00 | 5.00 | 5.00 | 5.00 | 5.00 | 5.00 | 5.00 |
| Cetyl Alcohol | 2.00 | 2.00 | 2.00 | 2.00 | 2.00 | 2.00 | 2.00 | 2.00 |
| Cetyl Phosphate | 2.00 | 2.00 | 2.00 | 2.00 | 2.00 | 2.00 | 2.00 | 2.00 |
| Isopropyl Myristate | 10.00 | 10.00 | 10.00 | 10.00 | 10.00 | 10.00 | 10.00 | 10.00 |
| Tocopheryl Acetate | 0.30 | 0.30 | 0.30 | 0.30 | 0.30 | 0.30 | 0.30 | 0.30 |
| Almond Oil | 2.00 | 2.00 | 2.00 | 2.00 | 2.00 | 2.00 | 2.00 | 2.00 |
| BHT | 0.05 | 0.05 | 0.05 | 0.05 | 0.05 | 0.05 | 0.05 | 0.05 |
| Trichostatin A | - | 0.01 | - | - | - | - | - | - |
| Phenoxyethanol & Methylparaben & Ethylparaben & Propylparaben & Butylparaben & Isopropylparaben | 0.60 | 0.60 | 0.60 | 0.60 | 0.60 | 0.60 | 0.60 | 0.60 |
| Tromethamine | 0.90 | 0.90 | 0.90 | 0.90 | 0.90 | 0.90 | 0.90 | 0.90 |
| | Ad. 100 | Ad.100 | Ad.100 | Ad. 100 | Ad.100 | Ad.100 | Ad.100 | Ad. 100 |
| Water | | | | | | | | |
| D-Panthenol | 0.20 | 0.20 | 0.20 | 0.20 | 0.20 | 0.20 | 0.20 | 0.20 |
| Disodium EDTA | 0.10 | 0.10 | 0.10 | 0.10 | 0.10 | 0.10 | 0.10 | 0.10 |
| Propylene Glycol | 4.00 | 4.00 | 4.00 | 4.00 | 4.00 | 4.00 | 4.00 | 4.00 |
| Polyacrylamide & C13-14 Isoparaffin & Laureth-7 | 2.00 | 2.00 | 2.00 | 2.00 | 2.00 | 2.00 | 2.00 | 2.00 |
| Phenylbutyric Acid | 0.50 | - | - | - | - | - | - | - |
| 4-Phenylbutyric Hydroxamic Acid | - | - | 0.20 | - | - | - | - | - |
| 3-Cyclohexyl-N-hydroxy-propionamide | - | - | - | 0.10 | - | - | - | - |
| 2-Cyclohexyl-N-hydroxy-acetamide | - | - | - | - | 0.10 | - | - | - |
| N-(5-Hydroxycarbamoyl-pentyl)4-methoxy-benzamide | - | - | - | - | - | 0.10 | - | - |
| N-Hydroxy-4-[(4-phenyl-butyrylamino)-methyl]-benzamide | - | - | - | - | - | - | 0.10 | - |
| Octanedioic acid bis-hydroxyamide | - | - | - | - | - | - | - | 0.10 |
| Retinol 15D (Caprylic/Capric Triglyceride & Retinol) | 0.50 | 0.50 | - | - | 0.50 | 0.50 | 0.50 | 0.50 |
| | - | - | 0.10 | 0.10 | - | - | - | - |
| *Retinol Acetate 2.8 M1* | | | | | | | | |
| | q.s. | q.s. | q.s. | q.s. | q.s. | q.s. | q.s. | q.s. |
| *Triethanolamine* | | | | | | | | |

**Example 17 - 24 Hair loss sera (examples 18 to 24 are reference examples)**

| **Formulation No Ingredients** | **17 % (w/w)** | **18 % (wlw)** | **19 % (w/w)** | **20 % (w/w)** | **21 % (w/w)** | **22 % (w/w)** | **23 % (w/w)** | **24 % (w/w)** |
|---|---|---|---|---|---|---|---|---|
| Water | Ad.100 | Ad.100 | Ad. 100 | Ad. 100 | Ad.100 | Ad.100 | Ad.100 | Ad.100 |
| Ethanol | 8.00 | 8.00 | 8.00 | 8.00 | 8.00 | 8.00 | 8.00 | 8.00 |
| Isopropanol | 4.00 | 4.00 | 4.00 | 4.00 | 4.00 | 4.00 | 4.00 | 4.00 |
| Propylene Glycol | 5.00 | 5.00 | 5.00 | 5.00 | 5.00 | 5.00 | 5.00 | 5.00 |
| D-Panthenol | 0.20 | 0.20 | 0.20 | 0.20 | 0.20 | 0.20 | 0.20 | 0.20 |
| PEG-12 Dimethicone | 0.20 | 0.20 | 0.20 | 0.20 | 0.20 | 0.20 | 0.20 | 0.20 |
| PEG-40 Hydrogenated Castor Oil | 4.00 | 4.00 | 4.00 | 4.00 | 4.00 | 4.00 | 4.00 | 4.00 |
| Phytantriol | 0.05 | 0.05 | 0.05 | 0.05 | 0.05 | 0.05 | 0.05 | 0.05 |
| Vitamin E Acetate | 0.10 | 0.10 | 0.10 | 0.10 | 0.10 | 0.10 | 0.10 | 0.10 |
| Trichostatin A | - | 0.02 | - | - | - | - | - | - |
| Phenylbutyric Acid | 0.50 | - | - | - | - | - | - | - |
| 4-Phenylbutyric Hydroxamic Acid | - | - | 0.20 | - | - | - | - | - |
| 3-Cyclohexyl-N-hydroxy-propionamide | - | - | - | 0.10 | - | - | - | - |
| 2-Cyclohexyl-N-hydroxy-acetamide | - | - | - | - | 0.10 | - | - | - |
| N-(5-Hydroxycarbamoyl-pentyl)-4-methoxy-benzamide | - | - | - | - | - | 0.10 | - | - |
| N-Hydroxy-4-[(4-phenyl-butyrylamino)-methyl]-benzamide | - | - | - | - | - | - | 0.10 | - |
| Octanedioic acid bis-hydroxyamide | - | - | - | - | - | - | - | 0.10 |
| Retinol 15D (Caprylic/Capric Triglyceride & Retinol) | 0.50 | 0.50 | - | - | 0.50 | 0.50 | 0.50 | 0.50 |
| | - | - | 0.10 | 0.10 | - | - | - | - |
| *Retinol Acetate 2.8 MI* | | | | | | | | |
| | q.s. | q.s. | q.s. | q.s. | q.s. | q.s. | q.s. | q.s. |
| *NaOH 10%* | | | | | | | | |

**Examples 25 - 32 Skin fortifier lotions (examples 26 to 32 are reference examples)**

| **Formulation No. Ingredients** | **25 % (w/w)** | **26 % (w/w)** | **27 % (w/w)** | **28 % (wlw)** | **29 % (w/w)** | **30 % (w/w)** | **31 % (wlw)** | **32 % (w/w)** |
|---|---|---|---|---|---|---|---|---|
| Water | Ad. 100 | Ad. 100 | Ad. 100 | Ad.100 | Ad. 100 | Ad. 100 | Ad. 100 | Ad. 100 |
| D- Panthenol | 0.05 | 0.05 | 0.05 | 0.05 | 0.05 | 0.05 | 0.05 | 0.05 |
| Sodium Ascorbyl Phosphate | 0.20 | 0.20 | 0.20 | 0.20 | 0.20 | 0.20 | 0.20 | 0.20 |
| Propylene Glycol | 5.00 | 5.00 | 5.00 | 5.00 | 5.00 | 5.00 | 5.00 | 5.00 |
| Acrylates/C10-30 Alkyl Acrylate Crosspolymer | 0.50 | 0.50 | 0.50 | 0.50 | 0.50 | 0.50 | 0.50 | 0.50 |
| Sodium Hydroxide 30% | 0.40 | 0.40 | 0.40 | 0.40 | 0.40 | 0.40 | 0.40 | 0.40 |
| Disodium EDTA | 0.10 | 0.10 | 0.10 | 0.10 | 0.10 | 0.10 | 0.10 | 0.10 |
| Squalane | 2.00 | 2.00 | 2.00 | 2.00 | 2.00 | 2.00 | 2.00 | 2.00 |
| Phenoxyethanol & Methylparaben & Ethylparaben & Propylparaben & Butylparaben & Isopropylparaben | 0.80 | 0.80 | 0.80 | 0.80 | 0.80 | 0.80 | 0.80 | 0.80 |
| Coco-Caprylate/Caprate | 4.00 | 4.00 | 4.00 | 4.00 | 4.00 | 4.00 | 4.00 | 4.00 |
| BHT | 0.05 | 0.05 | 0.05 | 0.05 | 0.05 | 0.05 | 0.05 | 0.05 |
| Tocopheryl Acetate | 0.10 | 0.10 | 0.10 | 0.10 | 0.10 | 0.10 | 0.10 | 0.10 |
| Cyclomethicone | 3.00 | 3.00 | 3.00 | 3.00 | 3.00 | 3.00 | 3.00 | 3.00 |
| Glycerin | 3.00 | 3.00 | 3.00 | 3.00 | 3.00 | 3.00 | 3.00 | 3.00 |
| Trichostatin A | - | 0.01 | - | - | - | - | - | - |
| Phenylbutyric Acid | 0.50 | - | - | - | - | - | - | - |
| 4-Phenylbutyric Hydroxamic Acid | - | - | 0.20 | - | - | - | - | - |
| 3-Cyclohexyl-N-hydroxy-propionamide | - | - | - | 0.10 | - | - | - | - |
| 2-Cyclohexyl-N-hydroxy-acetamide | - | - | - | - | 0.10 | - | - | - |
| | - | - | - | - | - | 0.10 | - | - |
| *N-(5-Hydroxycarbamoyl-pentyl)-4-methoxy-benzamide* | | | | | | | | |
| | - | - | - | - | - | - | 0.10 | - |
| *N-Hydroxy-4-[(4-phenyl-butyrylamino)-methyl]-benzamide* | | | | | | | | |
| | - | - | - | - | - | - | - | 0.10 |
| *Octanedioic acid bis-hydroxyamide* | | | | | | | | |
| | 0.50 | 0.50 | - | - | 0.50 | 0.50 | 0.50 | 0.50 |
| *Retinol 15D (Caprylic*/*Capric Triglyceride & Retinol)* | | | | | | | | |
| | - | - | 0.10 | 0.10 | - | - | - | - |
| *Retinol Acetate 2.8 MI* | | | | | | | | |
| *Sodium Hydroxide 10%* | q.s. | q.s. | q.s. | q.s. | q.s. | q.s. | q.s. | q.s. |

**Examples 33 - 40 Formulations to treat age spots (examples 34 to 40 are reference examples)**

| **Formulation No Ingredients** | **33 % (w/w)** | **34 % (w/w)** | **35 % (w/w)** | **36 % (w/w)** | **37 % (w/w)** | **38 % (w/w)** | **39 % (w/w)** | **40 % (w/w)** |
|---|---|---|---|---|---|---|---|---|
| Water | Ad.100 | Ad. 100 | Ad. 100. | Ad. 100. | Ad. 100 | Ad. 100. | Ad. 100 | Ad. 100 |
| Polyquatemium-10 | 0.10 | 0.10 | 0.10 | 0.10 | 0.10 | 0.10 | 0.10 | 0.10 |
| D- Panthenol | 0.50 | 0.50 | 0.50 | 0.50 | 0.50 | 0.50 | 0.50 | 0.50 |
| Sodium Ascorbyl Phosphate | 1.00 | 1.00 | 1.00 | 1.00 | 1.00 | 1.00 | 1.00 | 1.00 |
| Niacinamid | 0.50 | 0.50 | 0.50 | 0.50 | 0.50 | 0.50 | 0.50 | 0.50 |
| Propylene Glycol | 4.00 | 4.00 | 4.00 | 4.00 | 4.00 | 4.00 | 4.00 | 4.00 |
| Glycerin | 3.00 | 3.00 | 3.00 | 3.00 | 3.00 | 3.00 | 3.00 | 3.00 |
| PEG-12 Dimethicone | 0.20 | 0.20 | 0.20 | 0.20 | 0.20 | 0.20 | 0.20 | 0.20 |
| Disodium EDTA | 0.10 | 0.10 | 0.10 | 0.10 | 0.10 | 0.10 | 0.10 | 0.10 |
| Polysorbate 20 | 5.00 | 5.00 | 5.00 | 5.00 | 5.00 | 5.00 | 5.00 | 5.00 |
| Phenoxyethanol & Methylparaben & Ethylparaben & Propylparaben & Butylparaben & Isopropylparaben | 0.80 | 0.80 | 0.80 | 0.80 | 0.80 | 0.80 | 0.80 | 0.80 |
| Trichostatin A | - | 0.01 | - | - | - | - | - | - |
| Phenylbutyric Acid | 0.50 | - | - | - | - | - | - | - |
| 4-Phenylbutyric Hydroxamic Acid | - | - | 0.20 | - | | - | - | - |
| 3-Cyclohexyl-N-hydroxy-propionamide | - | - | - | 0.10 | - | - | - | - |
| | - | - | - | - | 0.10 | - | - | - |
| *2-Cyclohexyl-N-hydroxy-acetamide* | | | | | | | | |
| | - | - | - | - | - | 0.10 | - | - |
| *N-(5-Hydroxycarbamoyl-pentyl)-4-methoxy-benzamide* | | | | | | | | |
| | - | - | - | - | - | - | 0.10 | - |
| *N-Hydroxy-4-[(4-phenyl-butyrylamino)-methyl]-benzamide* | | | | | | | | |
| | - | - | - | - | - | - | - | 0.10 |
| *Octanedioic acid bis-hydroxyamide* | | | | | | | | |
| | 0.50 | 0.50 | - | - | 0.50 | 0.50 | 0.50 | 0.50 |
| *Retinol 15D (Caprylic*/*Capric Triglyceride & Retinol)* | | | | | | | | |
| | - | - | 0.10 | 0.10 | - | - | - | - |
| *Retinol Acetate 2.8 MI* | | | | | | | | |
| | q.s. | q.s. | q.s. | q.s. | q.s. | q.s. | q.s. | q.s. |
| *Sodium Hydroxide 10%* | | | | | | | | |

**Examples 41 - 48 Anti-cellulite formulations (examples 42 to 48 are reference examples)**

| **Formulation No. Ingredients** | **41 % (w/w)** | **42 % (w/w)** | **43 % (w/w)** | **44 % (w/w)** | **45 % (wlw)** | **46 % (w/w)** | **47 % (w/w)** | **48 % (w/w)** |
|---|---|---|---|---|---|---|---|---|
| | Ad. 100 | Ad. 100 | Ad. 100 | Ad. 100 | Ad.100 | Ad. 100 | Ad. 100 | Ad. 100 |
| Water | | | | | | | | |
| | 1.00 | 1.00 | 1.00 | 1.00 | 1.00 | 1.00 | 1.00 | 1.00 |
| Caffeine | | | | | | | | |
| D-Panthenol | 0.50 | 0.50 | 0.50 | 0.50 | 0.50 | 0.50 | 0.50 | 0.50 |
| Glycerin | 4.00 | 4.00 | 4.00 | 4.00 | 4.00 | 4.00 | 4.00 | 4.00 |
| Butylene Glycol | 2.00 | 2.00 | 2.00 | 2.00 | 2.00 | 2.00 | 2.00 | 2.00 |
| Acrylates/C10-30 Alkyl Acrylate Crosspolymer | 0.20 | 0.20 | 0.20 | 0.20 | 0.20 | 0.20 | 0.20 | 0.20 |
| Disodium EDETA | 0.10 | 0.10 | 0.10 | 0.10 | 0.10 | 0.10 | 0.10 | 0.10 |
| Arachidyl Alcohol & Behenyl Alcohol & Arachidyl Glucoside | 5.00 | 5.00 | 5.00 | 5.00 | 5.00 | 5.00 | 5.00 | 5.00 |
| Squalane | 2.00 | 2.00 | 2.00 | 2.00 | 2.00 | 2.00 | 2.00 | 2.00 |
| Mineral Oil | 4.00 | 4.00 | 4.00 | 4.00 | 4.00 | 4.00 | 4.00 | 4.00 |
| Phenoxyethanol & Methylparaben & Ethylparaben & Propylparaben & Butylparaben & Isopropylparaben | 0.80 | 0.80 | 0.80 | 0.80 | 0.80 | 0.80 | 0.80 | 0.80 |
| Isononyllsononanoate | 4.00 | 4.00 | 4.00 | 4.00 | 4.00 | 4.00 | 4.00 | 4.00 |
| Trichostatin A | - | 0.01 | - | - | - | - | - | - |
| BHT | 0.05 | 0.05 | 0.05 | 0.05 | 0.05 | 0.05 | 0.05 | 0.05 |
| Cetyl Alcohol | 2.00 | 2.00 | 2.00 | 2.00 | 2.00 | 2.00 | 2.00 | 2.00 |
| Dimethicone | 0.50 | 0.50 | 0.50 | 0.50 | 0.50 | 0.50 | 0.50 | 0.50 |
| Tocopheryl Acetate | 0.10 | 0.10 | 0.10 | 0.10 | 0.10 | 0.10 | 0.10 | 0.10 |
| Phenylbutyric Acid | 0.50 | - | - | - | - | - | - | - |
| 4-Phenylbutyric Hydroxamic Acid | - | - | 0.20 | - | - | - | - | - |
| 3-Cyclohexyl-N-hydroxy-propionamide | - | - | - | 0.10 | - | - | - | - |
| 2-Cyclohexyl-N-hydroxy-acetamide | - | - | - | - | 0.10 | - | - | - |
| | - | - | - | - | - | 0.10 | - | - |
| *N-(5-Hydroxycarbamoyl-pentyl)-4-methoxy-benzamide* | | | | | | | | |
| | - | - | - | - | - | - | 0.10 | - |
| *N-Hydroxy-4-[(4-phenyl-butyrylamino)-methyl]-benzamide* | | | | | | | | |
| | - | - | - | - | - | - | - | 0.10 |
| *Octanedioic acid bis-hydroxyamide* | | | | | | | | |
| | 0.50 | 0.50 | - | - | 0.50 | 0.50 | 0.50 | 0.50 |
| *Retinol 15D (Caprylic*/*Capric Triglyceride & Retinol)* | | | | | | | | |
| | - | - | 0.10 | 0.10 | - | - | - | - |
| *Retinol Acetate 2.8 MI* | | | | | | | | |
| | q.s. | q.s. | q.s. | q.s. | q.s. | q.s. | q.s. | q.s. |
| *Triethanolamine* | | | | | | | | |

**Examples 49 - 56 Skin repair formulations (examples 50 to 56 are reference examples)**

| **Formulation Nr Ingredients** | **49 % (w/w)** | **50 % (w/w)** | **51 % (wlw)** | **52 % (w/w)** | **53 % (w/w)** | **54 % (w/w)** | **55 % (w/w)** | **56 % (w/w)** |
|---|---|---|---|---|---|---|---|---|
| Polyglyceryl-2 Dipolyhydroxystearate | 4.00 | 4.00 | 4.00 | 4.00 | 4.00 | 4.00 | 4.00 | 4.00 |
| Polyglyceryl-3 Diisostearate | 2.00 | 2.00 | 2.00 | 2.00 | 2.00 | 2.00 | 2.00 | 2.00 |
| | 2.00 | 2.00 | 2.00 | 2.00 | 2.00 | 2.00 | 2.00 | 2.00 |
| Beeswax | | | | | | | | |
| Zinc Stearate | 2.00 | 2.00 | 2.00 | 2.00 | 2.00 | 2.00 | 2.00 | 2.00 |
| Caprylic/Capric Triglyceride | 3.00 | 3.00 | 3.00 | 3.00 | 3.00 | 3.00 | 3.00 | 3.00 |
| Cetearyl Isononanoate | 8.00 | 8.00 | 8.00 | 8.00 | 8.00 | 8.00 | 8.00 | 8.00 |
| Dicaprylyl Ether | 5.00 | 5.00 | 5.00 | 5.00 | 5.00 | 5.00 | 5.00 | 5.00 |
| Trichostatin A | - | 0.015 | - | - | - | - | - | - |
| BHT | 0.05 | 0.05 | 0.05 | 0.05 | 0.05 | 0.05 | 0.05 | 0.05 |
| Phenoxyethanol & Methylparaben & Ethylparaben & Propylparaben & Butylparaben & Isopropylparaben | 0.60 | 0.60 | 0.60 | 0.60 | 0.60 | 0.60 | 0.60 | 0.60 |
| | Ad.100 | Ad.100 | Ad. 100 | Ad. 100 | Ad.100 | Ad.100 | Ad.100 | Ad. 100 |
| Water | | | | | | | | |
| D-Panthenol | 0.20 | 0.20 | 0.20 | 0.20 | 0.20 | 0.20 | 0.20 | 0.20 |
| Disodium EDTA | 0.10 | 0.10 | 0.10 | 0.10 | 0.10 | 0.10 | 0.10 | 0.10 |
| Propylene Glycol | 4.00 | 4.00 | 4.00 | 4.00 | 4.00 | 4.00 | 4.00 | 4.00 |
| Phenylbutyric Acid | 0.45 | - | - | - | - | - | - | - |
| 4-Phenylbutyric Hydroxamic Acid | - | - | 0.20 | - | - | - | - | - |
| 3-Cyclohexyl-N-hydroxy-propionamide | - | - | - | 0.10 | - | - | - | - |
| 2-Cyclohexyl-N-hydroxy-acetamide | - | - | - | - | 0.10 | - | - | - |
| N-(5-Hydroxycarbamoyl-pentyl)-4-methoxy-benzamide | - | - | - | - | - | 0.10 | - | - |
| N-Hydroxy-4-[(4-phenyl-butyrylamino)-methyl]-benzamide | - | - | - | - | - | - | 0.10 | - |
| | - | - | - | - | - | - | - | 0.10 |
| **Octanedioic acid bis-hydroxyamide** | | | | | | | | |
| Retinol 15D (Caprylic/Capric Triglyceride & Retinol) | 0.50 | 0.50 | - | - | 0.50 | 0.50 | 0.50 | 0.50 |
| | - | - | 0.10 | 0.10 | - | - | - | - |
| *Retinol Acetate 2.8 MI* | | | | | | | | |

### Reference Example 57: Biological synergism between Retinol and Trichostatin A on human keratinocytes

This example illustrates how the HDAC inhibitor Trichostatin A and Retinol work synergistically. An in vitro cell culture of human primary keratinocytes has been chosen as test system. This *in vitro* cell culture model is useful to study differentiation processes and is able to simulate the *in vivo* situation in human skin (Poumay et al., 1999). To monitor differentiation processes *in vitro* and *in vivo* transglutaminase 1 is a well known marker molecule (Polakowska et al., 1999).

### Culture of epidermal keratinocytes

Epidermal keratinocytes were isolated from human foreskin biopsies and cultured in keratinocyte serum free medium (KSFM made by GIBCO) in a growth chamber with 37°C and 5 % CO2. At the second passage, cells were transferred to 6 well plates and allowed to reach approximately 50% surface confluence.

### Expression of transglutaminase 1 (TG1) by differentiating keratinocytes

Retinol and TSA were solubilized in ethanol or ethanol/tetrahydrofuran respectively. Retinol solutions were handled under yellow light conditions only. When keratinocyte cultures had reached the appropriate confluence, the KSFM medium was supplemented with 1.3 mM calcium, in order to induce keratinocyte differentiation and thus also induce TG1 expression, and treatment was started with either retinol in concentrations of 1x10-10 M and for TSA at 10-9 M, or the two substances in combination. For every sample, medium and treatment substances were changed twice daily.

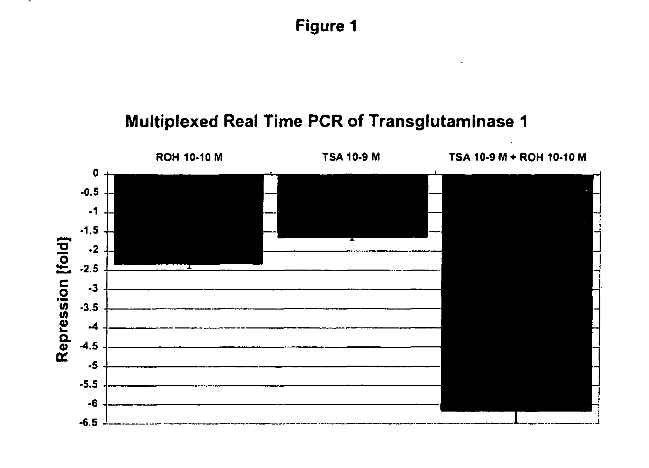

Seventy-two hours after the beginning of the treatment, cells were harvested and the RNA extracted. RNA was reverse transcribed into cDNA. Relative quantification of TG1 mRNA transcript levels in control versus treatment cultures were determined using multiplexed real time PCR analysis. Repression level is given as a multiplicator relative to the non-treated test cell. The result is shown in figure 1, ROH designates retinol.

Retinol and TSA alone showed only weak modulation of the terminal differentiation process in epidermal keratinocytes. Surprisingly in combination an impressive synergism emerges. The differentiation marker TG1 in human epidermal keratinocytes was more than 6-fold downregulated.

The enzyme transglutaminase 1 is a well-known and accepted differentiation marker in human epidermal cells. Cultures with keratinocytes expressing this marker are a model to simulate the differentiation process of the skin. Slowing this process down results in an improved epidermal thickness. According to the invention a HDAC inhibitor and a retinoid slowed down the process with maximum effect when applied together.

### Assessment on collagen production, melanogenesis and lipostasis

Synergism leading to improved collagen production can be assessed with cultured fibroblasts. Influences on melanogenesis can be investigated with cultured melanocytes and changes in the balance of lipolysis and lipogenesis (lipostasis) can be detected with cultured adipocytes.

### Assessment of accelerated hair growth and protection hair growth

The ability of the compounds and compositions of the present invention to stimulate or protect hair growth can be investigated with a mouse model described for example in WO 9817273. Instead of using Cyclophosphamide (Neostar, Pharmacia) to damage hair follicle Mitomycin or Methotrexate can be used. It is also possible to detect hair growth acceleration with newborn mice. They have a synchronized hair cycle and approximately after 3 weeks all hair follicles go into the telogen phase. Then the animals are treated and it is evaluated how fast and to what extend the hair is growing. Similar tests using *in vitro* or *in vivo* setups can also be found in J. Invest. Dermato. symposium proceedings 3rd Int. Meeting of Hair Research Societies, 8/1, p.39 - 45 (2003).

It also is possible to perform a clinical study including males suffering from alopecia using the TrichoScan analysis tool described in R. Hoffmann, J. Invest. Dermato. symposium proceedings 3rd Int. Meeting of Hair Research Societies, 8/1, p.109-115 (2003),

## Claims

1. Topical composition comprising at least one HDAC inhibitor in combination with a retinoid and a cosmetically or pharmaceutically acceptable excipient or diluent, wherein the HDAC inhibitor is a compound of the formula 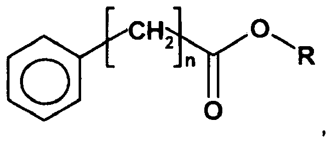 wherein
R is a C₁-C₃₀ hydrocarbon residue and
n is an integer of 1 to 5.

2. Composition according to claim 1, wherein R is a C₄-C₂₀ alkyl group or a C₄-C₂₀ alkenyl group.

3. Composition according to any of claims 1 to 2, wherein the retinoid is a compound of the formula 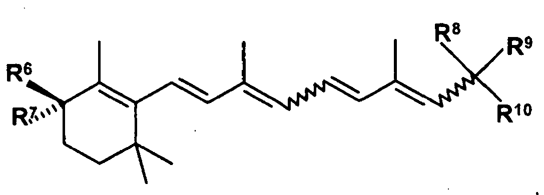
wherein R⁶ and R⁷ are independently of each other hydrogen or hydroxy or R⁶ and R⁷ together form an oxygen atom
R⁸ and R⁹ are hydrogen or R⁸ and R⁹ together form an oxygen atom
R¹⁰ is hydrogen, hydroxy or a residue OR¹¹
R¹¹ is C₁-C₂₀ alkyl (preferably C₁-C₆ alkyl), C₂-C₂₀ alkenyl (preferably C₂-C₆ alkenyl), C₆-C₂₀ aryl, C₇-C₂₀ alkylaryl or a residue -C(O)-R¹²
R¹² is C₁-C₂₀ alkyl (preferably C₁-C₆ alkyl) or C₂-C₂₀ alkenyl (preferably C₂-C₆ alkenyl).

4. Composition according to any of claims 1 to 3, wherein the composition contains the HDAC inhibitor in a concentration of 0.001 to 50 wt.-%, based on the weight of the composition.

5. Composition according to claim 4, wherein the HDAC inhibitor is present in a concentration of 0.01 to 1 wt.-%, based on the weight of the composition.

6. Composition according to any of claims 1 to 5, wherein the retinoid is present in a concentration of 0.001 to 50 wt.-%, based on the weight of the composition.

7. Composition according to claim 6, wherein the retinoid is present in a concentration of 0.1 to 15 wt.-%, based on the weight of the composition.

8. Composition according to any of claims 1 to 7, wherein the ratio of the HDAC inhibitor to the retinoid is from 30:1 to 1:30, based on the weight.

9. Composition according to any of claims 1 to 8, which is a cosmetic composition.

10. Use of a combination of an HDAC inhibitor and a retinoid for providing a cosmetic effect selected from treating and preventing skin wrinkles, ameliorating the effects of aging of the skin and thickening of the epidermis, wherein the HDAC inhibitor is a compound of the formula 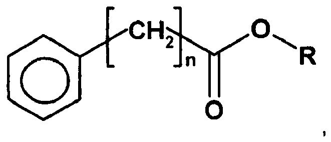 wherein
R is hydrogen or a C₁-C₃₀ hydrocarbon residue and
n is an integer of 1 to 5.

## Patentansprüche

1. Topische Zusammensetzung, umfassend mindestens einen HDAC-Inhibitor in Kombination mit einem Retinoid und einen kosmetisch oder pharmazeutisch akzeptablen Hilfsstoff oder ein kosmetisch oder pharmazeutisch akzeptables Verdünnungsmittel, wobei der HDAC-Inhibitor eine Verbindung der Formel 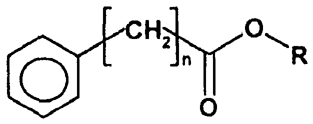 ist, wobei
R ein C₁-C₃₀-Kohlenwasserstoffrest ist und
n eine ganze Zahl von 1 bis 5 ist.

2. Zusammensetzung nach Anspruch 1, wobei R eine C₄-C₂₀-Alkylgruppe oder eine C₄-C₂₀-Alkenylgruppe ist.

3. Zusammensetzung nach einem der Ansprüche 1 bis 2, wobei das Retinoid eine Verbindung der Formel 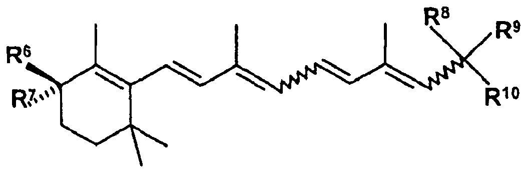
ist, wobei R⁶ und R⁷ unabhängig voneinander Wasserstoff oder Hydroxy sind oder R⁶ und R⁷ zusammen ein Sauerstoffatom bilden,
R⁸ und R⁹ Wasserstoff sind oder R⁸ und R⁹ zusammen ein Sauerstoffatom bilden,
R¹⁰ Wasserstoff, Hydroxy oder ein Rest OR¹¹ ist;
R¹¹ C₁-C₂₀-Alkyl (bevorzugt C₁-C₆-Alkyl), C₂-C₂₀-Alkenyl (bevorzugt C₂-C₆-Alkenyl), C₆-C₂o-Aryl, C₇-C₂₀-Alkylaryl oder ein Rest -C(O)-R¹² ist;
R¹² C₁-C₂₀-Alkyl (bevorzugt C₁-C₆-Alkyl) oder C₂-C₂₀-Alkenyl (bevorzugt C₂-C₆-Alkenyl) ist.

4. Zusammensetzung nach einem der Ansprüche 1 bis 3, wobei die Zusammensetzung den HDAC-Inhibitor in einer Konzentration von 0,001 bis 50 Gew.-%, basierend auf dem Gewicht der Zusammensetzung, enthält.

5. Zusammensetzung nach Anspruch 4, wobei der HDAC-Inhibitor in einer Konzentration von 0,01 bis 1 Gew.-%, basierend auf dem Gewicht der Zusammensetzung, vorliegt.

6. Zusammensetzung nach einem der Ansprüche 1 bis 5, wobei das Retinoid in einer Konzentration von 0,001 bis 50 Gew.-%, basierend auf dem Gewicht der Zusammensetzung, vorliegt.

7. Zusammensetzung nach Anspruch 6, wobei das Retinoid in einer Konzentration von 0,1 bis 15 Gew.-%, basierend auf dem Gewicht der Zusammensetzung, vorliegt.

8. Zusammensetzung nach einem der Ansprüche 1 bis 7, wobei das Verhältnis des HDAC-Inhibitors zu dem Retinoid 30 : 1 bis 1 : 30, basierend auf dem Gewicht, beträgt.

9. Zusammensetzung nach einem der Ansprüche 1 bis 8, die eine kosmetische Zusammensetzung ist.

10. Verwendung einer Kombination aus einem HDAC-Inhibitor und einem Retinoid zur Bereitstellung einer kosmetischen Wirkung, ausgewählt aus der Behandlung und Vorbeugung von Hautfalten, Bessern der Auswirkungen des Alterns der Haut und Verdicken der Epidermis, wobei der HDAC-Inhibitor eine Verbindung der Formel 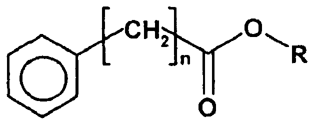 ist, wobei
R Wasserstoff oder ein C₁-C₃₀-Kohlenwasserstoffrest ist und n eine ganze Zahl von 1 bis 5 ist.

## Revendications

1. Composition topique comprenant au moins un inhibiteur HDAC en combinaison avec un retinoïde et un excipient ou diluant cosmétiquement ou pharmaceutiquement acceptable, dans laquelle l'inhibiteur HDAC est un composé ayant pour formule 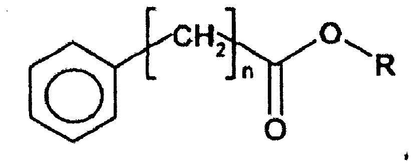 dans laquelle
R est un résidu hydrocarboné C₁-C₃₀ et
n est un entier de 1 à 5.

2. Composition selon la revendication 1, dans laquelle R est un groupe alkyle C₄-C₂₀ ou un groupe alkényle C₄-C₂₀.

3. Composition selon la revendication 1 ou 2, dans laquelle le rétinoïde est un composé ayant pour formule 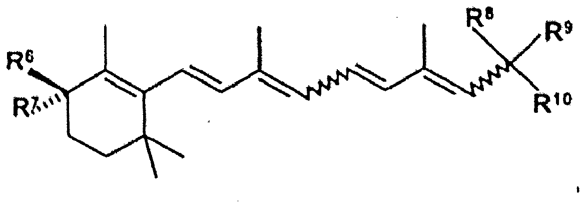
dans laquelle R⁶ et R⁷ sont, indépendamment l'un de l'autre, un hydrogène ou un hydroxy, ou R⁶ et R⁷ forment ensemble un atome d'oxygène
R⁸ et R⁹ sont un hydrogène ou R⁸ et R⁹ forment ensemble un atome d'oxygène
R¹⁰ est un hydrogène, un hydroxy ou un résidu OR¹¹
R¹¹ est un alkyle C₁-C₂₀ (de préférence un alkyle C₁-C₆), un alkényle C₂-C₂₀ (de préférence un alkényle C₂-C₆), un aryle C₆-C₂₀, un alkylaryle C₇-C₂₀ ou un résidu -C(O)-R¹²
R¹² est un alkyle C₁-C₂₀ (de préférence un alkyle C₁-C₆) ou un alkényle C₂-C₂₀ (de préférence un alkényle C₂-C₆).

4. Composition selon l'une des revendications 1 à 3, dans laquelle la composition contient l'inhibiteur HDAC dans une concentration de 0.001 à 50 % en poids, basé sur le poids de la composition.

5. Composition selon la revendication 4, dans laquelle l'inhibiteur HDAC est présent dans une concentration de 0.01 à 1 % en poids, basé sur le poids de la composition.

6. Composition selon l'une quelconque des revendications 1 à 5, dans laquelle le rétinoïde est présent à une concentration de 0.001 à 50 % en poids, basé sur le poids de la composition.

7. Composition selon la revendication 6, dans laquelle le rétinoïde est présent à une concentration de 0.1 à 15 % en poids, basé sur le poids de la composition.

8. Composition selon l'une quelconque des revendications 1 à 7, dans laquelle le ratio d'inhibiteur HDAC par rapport au rétinoïde est de 30:1 à 1:30, basé sur la masse.

9. Composition selon l'une quelconque des revendications 1 à 8, qui est une composition cosmétique.

10. Utilisation d'une combinaison d'un inhibiteur de HDAC et d'un rétinoïde pour pourvoir un effet cosmétique choisi parmi le traitement et la prévention de rides sur la peau, l'amélioration des effets du vieillissement de la peau et l'épaississement de l'épiderme, dans laquelle l'inhibiteur de HDAC est un composé ayant pour formule 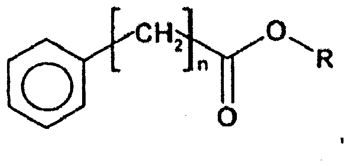 dans laquelle
R est un hydrogène ou un résidu hydrocarboné C₁-C₃₀ et n est un entier de 1 à 5.
